Europäisches Patentamt

European Patent Office

Office européen des brevets

(11) Publication number: **0 096 905**
**B1**

# EUROPEAN PATENT SPECIFICATION

(45) Date of publication of patent specification: 23.04.86

(21) Application number: 83200613.4

(22) Date of filing: 29.04.83

(51) Int. Cl.⁴: **C 07 C 47/19,** C 07 C 45/68,
C 07 C 31/20, C 07 C 29/14

(54) Process for the preparation of glycol aldehyde.

(30) Priority: 16.06.82 GB 8217453

(43) Date of publication of application:
**28.12.83 Bulletin 83/52**

(45) Publication of the grant of the patent:
**23.04.86 Bulletin 86/17**

(84) Designated Contracting States:
**BE DE FR GB IT NL**

(56) References cited:
**EP-A-0 002 908**
**EP-A-0 046 680**
**EP-A-0 061 791**
**US-A-4 200 765**
**US-A-4 291 179**

**JOURNAL OF ORGANOMETALLIC
CHEMISTRY, vol. 194, no. 1, 1980, Lausanne, A.
SPENCER "Hydroformylation of formaldehyde
catalysed by rhodium complexes", pages 113-
123**

(73) Proprietor: SHELL INTERNATIONALE
RESEARCH MAATSCHAPPIJ B.V.
Carel van Bylandtlaan 30
NL-2596 HR Den Haag (NL)

(72) Inventor: **Drent, Eit**
**Badhuisweg 3**
**NL-1031 CM Amsterdam (NL)**

(74) Representative: **Aalbers, Onno et al**
**P.O. Box 302**
**NL-2501 CH The Hague (NL)**

(56) References cited:
**Chemical Abstracts, vol. 97, no. 17, 25 October
1982, Columbus, Ohio, USA, T. SUZUKI
"Kinetics of rhodium-catalyzed
hydroformylation of formaldehyde", page 621,
column 2, abstract no. 144126w**

The file contains technical information
submitted after the application was filed and
not included in this specification

Courier Press, Leamington Spa, England.

## Description

This invention relates to a process for the preparation of glycol aldehyde by reacting formaldehyde with hydrogen and carbon monoxide in the presence of a catalyst system derived from a rhodium-containing catalyst precursor and/or a cobalt-containing catalyst precursor, an acid and a promoter XR₃, X representing P, As or Sb and each R representing a similar or dissimilar substituted or unsubstituted hydrocarbyl group.

Such a process for the preparation of glycol aldehyde, which is a useful intermediate for the preparation of ethylene glycol, is described in European Patent Application 0 061 791. As acid a strong protonic acid is used. A side reaction in this known process is the formation of methanol, as a result of hydrogenation of formaldehyde.

European patent application 0 002 908 describes a process for the preparation of glycol aldehyde by reacting formaldehyde with hydrogen and carbon monoxide in the presence of a catalyst system derived from a rhodium-containing catalyst and a phosphine ligand. The rhodium-containing catalyst may contain an acetic acid anion. This known process suffers from the disadvantage that much methanol is produced.

The Applicants have described and claimed in co-pending application 8109119 that the production of glycol aldehyde in this type of process, when using either a cobalt or a rhodium catalyst, can be improved by the addition to the reaction mixture of a catalytic amount of a strong protonic acid. In particular, the quantity of methanol formed can be much reduced.

It is known from U.S. patent specification 4,200,765 that protic solvents such as water, phenols and carboxylic acids can be used (primarily to facilitate hydrolysis of unwanted acetals coproduced), but the use of lower alkanoic acids such as acetic acid is clearly disadvantageous in that, although the reaction proceeds at a higher rate, lower yields of glycol aldehyde are produced, particularly when rather high amounts of such protic solvents are used. It is stated explicitly that protic solvents in significant amounts are usually avoided when optimum yields of glycol aldehyde and ethylene glycol and minimum yields of methanol are desired.

It has now been found that the presence of certain acids, in particular certain carboxylic acids can be used advantageously in the process for the preparation of glycol aldehyde from formaldehyde, carbon monoxide and hydrogen when use is made of promoters XR₃ wherein at least one of R represents an aryl group containing one or more electron-withdrawing groups. It is remarkable that only the combined presence of certain acids as defined hereinafter and promoters containing aryl groups substituted in such a way that in total an electron-withdrawing effect results, causes the production of glycol aldehyde with both very high yield (well over 80%) and very high selectivity (>90%). In the absence of an acid the yield drops to about 5% and in the presence of unsubstituted XR₃ promoters such as triphenylphosphine both the selectivity and the yield are moderate at best. Admittedly, the use of substituted triphenyl-phosphines has been proposed before but there is nothing in that proposal to suggest the use of specifically substituted arylphosphines together with carboxylic acids, let alone the unexpected and substantial improvement in yield and selectivity.

The present invention therefore provides a process for the preparation of glycol aldehyde by reacting formaldehyde with hydrogen and carbon monoxide in the presence of a catalyst system derived from a rhodium-containing catalyst precursor and/or a cobalt-containing catalyst precursor, an acid and a promoter XR₃, X representing P, As or Sb and each R representing a similar or dissimilar substituted or unsubstituted hydrocarbyl group, characterized in that the reaction is carried out in the presence of an acid having a pK$_a$ ≥ 3.5 and that at least one R in the formula XR₃ represents an aryl group containing one or more electron-withdrawing groups.

The catalyst precursor used in the process according to the invention comprises rhodium and/or cobalt in any form generally used in catalytic reactions. The precursor may for example be a salt of rhodium or cobalt with a mineral acid, for example a halide, nitrate or sulphate, or with an organic acid, for example a carboxylate having up to 20 carbon atoms, especially alkanoate, such as an acetate. Alternatively, the metal may be in zerovalent form, optionally complexed by ligand such as (substituted) phosphine ligands, carbon monoxide or acetylacetonates. Frequently both anions and uncharged ligands are present, e.g. as in [Rh.Cl(CO)₂]₂. The precise form of the active catalyst in the process of the invention is not known; in some cases it is believed that the rhodium- or cobalt-containing catalyst precursor added to the reaction mixture will itself function directly as a catalyst, in other cases it is believed that is will be converted into an active form *in situ*.

The quantity of rhodium or cobalt present in the reaction mixture is generally determined by economic considerations. Quantities of rhodium plus cobalt of between 0.001 to 10%, especially 0.01 to 5%, calculated as gram atoms of metal per mole of formaldehyde used as feedstock, are generally suitable. Generally rhodium is more active as a catalyst than cobalt, but the use of cobalt may be desirable because of its relatively low cost. In certain cases, a catalyst system containing both rhodium and cobalt may be useful.

The process according to the present invention has to be carried out in the presence of acids having a pK$_a$ of at least 3.5 at room temperature, preferably between 3.5 and 11. A suitable class of acids comprises aliphatic monocarboxylic acids such as formic acid, acetic acid, propionic acid, butyric acid, isobutryric acid, caproic acid, isocaproic acid, pivalic acid and valeric acid; aliphatic dicarboxylic acids having at least two carbon atoms between the carboxylic moieties such as adipic acid and succinic acid; aromatic carboxylic acids such as benzoic acid, α-naphthoic acid, β-naphthoic acid, phthalic acid, cis-cinnamic acid and trans-cinnamic acid as well as various substituted carboxylic acids having a $pK_a$ of at least 3.5 such as acetoacetic acid, p-chlorobenzoic acid and m-aminobenzoic acid; and unsaturated carboxylic acids such as acrylic acid, methacrylic acid and trans-crotonic acid. Preference is given to aliphatic monocarboxylic acids such as formic acid, acetic acid, propionic acid and the butyric acids; in particular good results have been obtained using acetic acid. It should be noted that carboxylic acids containing substituents which are susceptible to reaction with carbon monoxide and/or hydrogen can be used as long as the acid function therein is maintained.

Another class of suitable acids comprises partially esterified acids having a $pK_a$ of at least 3.5 at room temperature. The partially esterified acids may comprise both inorganic and organic acids. Examples of partially esterified inorganic acids comprise mono- and di-alkyl or di-aryl substituted phosphoric acids such as dimethyl phosphate, diethyl phosphate, diphenyl phosphate, methyl ethyl phosphate and methyl phenyl phosphate. Also partially esterified boric and tetraboric acids can be used. Examples of partially esterified organic acids comprise the monoesters of aliphatic dicarboxylic acids having at least two carbon atoms between the carboxyl moieties such as methyl adipate, ethyl succinate, t-butyl adipate and phenyl succinate as well as the partially esterified aromatic polycarboxylic acids such as methyl phthalate, ethyl phthalate, t-buyl-phthalate, methyl terephthalate and ethyl terephthalate.

A further class of suitable acids having a $pK_a$ (or the equivalent thereof) of at least 3.5 at room temperature comprises solid organic acids or solid partially esterified polyacids which are of the macroreticular type, i.e. acids bound to or forming part of a polymeric structure. Examples of such type of compounds comprise the so-called Duolites such as Duolite $C434H^+$, Duolite $C436H^+$ and Duolite $C464H^+$. The word "Duolite" is a Registered Trade Mark. Also other solid acids which are known in the art can be used.

Also phenolic compounds having an aromatic carbon hydroxyl bond can be used as acids in the process according to the present invention. Examples comprise phenol, α-naphthol, β-naphthol; cresols such as hydrochinone, resorcinol, and pyrocatechol; monohalophenols such as m-chlorophenol, m-bromophenol, p-chlorophenol, p-bromophenol; dihalophenols such as 2,3-dichlorophenol; nitrophenols and dinitrophenols such as 2,4-dinitrophenol and 3,6-dinitrophenol. Good results have been obtained using phenol as an example of phenolic compounds which can be used advantageously.

The amount of acid having a $pK_a$ of at least 3.5 is not very critical and may vary between rather wide limits provided that one or more promoters according to the general formula $XR_3$ are also present. Acids in an amount of up to 50%v on total (liquid) volume can be used, preference being given to acids in an amount between 5 and 30%v, in particular between 5 and 20%v.

The formaldehyde starting material may be introduced into the reaction zone in any suitable form, and it may be generated *in situ*. Paraformaldehyde is a convenient source. Commercial formaldehyde often contains varying quantities of either methanol or water, depending on how the material has been synthesised, and the process according to the invention can be carried out successfully using such feedstocks.

The molar ratio of the carbon monoxide to hydrogen supplied to the system is not critical and may vary over a wide range, for example 5:95 to 95:5, preferably 30:70 to 80:20. It is generally preferred to use a gas stream in which the molar ratio of $CO:H_2$ is at least 1:1, since under such conditions the hydrogenation of formaldehyde to methanol is minimized. The reaction is conducted under pressure, for example at a pressure in the range of from 5 to 200, especially 25 to 80, bars. Higher pressures may of course be used, but are generally uneconomical, and it is one of the advantages of the present invention that it enables relatively low pressures to be used. Inert gases may also be present in the gas stream, but as this leads to an increase in total pressure it is generally undesirable. The reaction is preferably conducted at a temperature in the range of from 30 to 200°C, especially 50 to 130°C. The use of a temperature as low as possible commensurate with the desired rate of reaction is preferred, since at much higher temperatures the glycol aldehyde product tends to polymerize.

The process according to the invention is suitably carried out in the additional presence of a solvent. Details of suitable solvents for reactions of this kind may be found in the prior art indicated hereinbefore. Solvents having multiple bonds from carbon to other atoms, for example as in nitriles or pyridine, are generally suitable. N,N-disubstituted amides have proved to be especially suitable solvents, optionally in admixture with cosolvents. If it is desired to extract the glycol aldehyde product from the reaction mixture using water, it is convenient to use a water-immiscible amide as solvent. Suitable water-immiscible amides are those containing long chain alkyl moieties. Alternatively, if an amide which is wholly or partially miscible with water is used, for example N,N-dimethylformamide, N,N-dimethylacetamide or a cyclic amide such as N-methyl-pyrrolidone, it may be convenient to use a water-immiscible co-solvent such as benzene. In this case, extraction by water removes the glycol aldehyde together with at least some of the amide, leaving the rhodium or cobalt in solution in the hydrophobic co-solvent.

Examples of compounds according to the general formula $XR_3$ are those wherein X represents a phosphorus atom and at least one R represents an aryl group containing one or more electron-withdrawing groups in the ortho, metha or para positions. Examples of electron-withdrawing groups comprise halogen moieties, tri(halomethyl) groups, nitro groups and alkoxy or aryloxy groups. Preference is given to compounds according to the general formula $XR_3$ wherein each group R is similar and contains at least one electron-withdrawing group. Examples of such compounds comprise tri(p-fluorophenyl)phosphine, tri(p-chlorophenyl)phosphine, tri(p-bromophenyl)phosphine, tri(p-iodophenyl)phosphine, tri(m-fluorophenyl)phosphine, tri(m-iodophenyl)phosphine, tri(o-chlorophenyl) phosphine, tri(o-bromophenyl)phosphine, tri(p-nitrophenyl)phosphine, tri(m-nitrophenylphosphine, tri(m-methoxyphenyl)phosphine, tri(m-ethoxyphenyl)phosphine, tri(m-t.butoxyphenyl)phosphine and tri(m-phenoxyphenyl)phosphine. Very good results have been obtained using tri(p-chlorophenyl)phosphine and tri(p-bromophenyl)phosphine.

It is also possible to use compounds according to the general formula $XR_3$ wherein one or two of R represent a group $(CH_2)_nXR_2$ wherein n is an integer of up to 6 and X and R are as defined hereinbefore. Examples of such compounds include bis-(tetra-p-chlorophenyl)phosphinomethane, bis(tetra-p-bromophenyl)phosphinoethane and bis(di-p-chlorophenyl) (diphenyl)phosphinopropane.

It should be noted that two or more electron-withdrawing substituents may be present in the hydrocarbyl or aryl groups such as in the 3,4-dichlorophenyl group, the 3-fluoro-4-chlorophenyl group or the 2,6-dinitro-4-(trifluoromethyl)phenyl group. It is also possible that the aryl groups contain further substituents which exert an electron-donating effect provided that the overall effect of the substituents in the aryl group results in an electron-withdrawing effect; in other words that the Hammett substituent constant σ, or the combined values of the respective Hammett σ-values, as the case may be, is at least+0.1 (see, for reference, J. Hine McGraw-Hill Book Company, 1962, Second Edition, page 87).

The amount of promoter used is not critical. The ratio of promoter to catalyst is preferably in the range of from 1:1 to 100:1, especially 2:1 to 20:1, calculated as moles of promoter per gram atom of rhodium plus cobalt. When promoters are applied which contain two X moieties, smaller amounts thereof are already sufficient.

The main use of glycol aldehyde is its conversion to ethylene glycol by catalytic hydrogenation. Under certain reaction conditions, some or all of the glycol aldehyde prepared by the process according to the invention may be hydrogenated *in situ* over the rhodium or cobalt catalyst to produce ethylene glycol, and the present invention should be understood to include the production of ethylene glycol as just mentioned.

In general, however, reaction conditions which tend to favour the hydrogenation of glycol aldehyde immediately it is formed, tend also to favour the hydrogenation of the formaldehyde starting material to methanol. Usually therefore the highest overall yields of ethylene glycol are obtained by preparing glycol aldehyde under reaction conditions which minimize hydrogenation, and subsequently hydrogenating the glycol aldehyde product in a second reaction step.

The rhodium or cobalt catalyst systems used in the process of the present invention are relatively inefficient hydrogenation catalysts, and it is preferred to use a more active hydrogenation catalyst for the subsequent hydrogenation step. Such catalysts are well known: for example palladium, platinum or nickel catalysts, often in heterogeneous form, are commonly used. The selected hydrogenation catalyst may be added directly to the reaction mixture after completion of the preparation of glycol aldehyde with no work-up procedure and gaseous hydrogen introduced. Hydrogen gas which is free from substantial quantities of carbon monoxide is of course a preferred reactant when using a hydrogenation catalyst when is poisoned by carbon monoxide. Alternatively, the reaction mixture resulting from the preparation of glycol aldehyde may be worked up before the glycol aldehyde is hdrogenated. For example the glycol aldehyde may be extracted using a suitable solvent. As described above, water is a convenient extractant. A further convenient extractant is ethylene glycol itself. The resulting solution may then be hydrogenated in conventional manner.

The following Examples illustrate the invention.

## Examples

All the Examples were carried out using the following general method. A Hastelloy C (Trade Mark) 300 ml magnet-driven autoclave was charged with 0.25 mol formaldehyde in the form of paraformaldehyde, 50 ml of the chosen solvent and the necessary catalyst components. The autoclave was then flushed with carbon monoxide and pressurized to a working pressure of 60 bars with a 1:1 molar carbon monoxide/hydrogen mixture. The pressure was maintained throughout the reaction by feeding in the $CO/H_2$ mixture as required. After the required reaction temperature and pressure had been maintained for the required reaction time, the contents of the autoclave were cooled and analysed using gas-liquid chromatography.

The following abbreviations have been used: DMA — dimethylacetamide; acac — the acetylacetonate ligand. The selectivity of the reaction is calculated as:

$$\frac{\text{moles glycol aldehyde}}{\text{moles glycol aldehyde + methanol}} \times 100\%$$

### Example 1

An experiment was carried out using 0.5 mmol $Rh(acac)-(CO)_2$, 2.5 mmol tri(p-chlorophenyl)phosphine, DMA and acetic acid (1 ml). The reaction was carried out at 80°C for a period of 3.5 h. The selectivity in %m as defined hereinbefore amounted to 88% and the yield of glycol aldehyde calculated on formaldehyde intake amounted to 62%.

### Example 2

The experiment described in Example 1 was repeated using 2.5 ml acetic acid. The reaction was carried out at 88°C for a period of 4 h. The selectivity amounted to 88%m and the yield of glycol aldehyde calculated on formaldehyde intake amounted to 82%.

### Example 3

The experiment described in the previous Example was repeated using double the amount of acetic acid (5 ml). The reaction was carried out for a period of 3.5 h. The selectivity amounted to 94%m and the yield, calculated on formaldehyde intake, amounted to 92%.

### Example 4

The experiment described in the previous Example was repeated using N-methylpyrrolidone as the solvent. The reaction was carried out at 80°C for a period of 3.5 h. The selectivity amounted to 89%m and the yield, calculated on formaldehyde intake, amounted to 70%.

### Example 5

The experiment described in Example 3 was repeated using 7.5 ml acetic acid. The reaction was carried out at 85°C for a period of 5 h. The selectivity amounted to 89%m and the yield, calculated on formaldehyde intake, amounted to 87%.

### Example 6

The experiment described in Example 3 was repeated using 2.5 mmol tri(p-bromophenyl)-phosphine. The reaction was carried out at 80°C for a period of 3.5 h. The selectivity amounted to 90%m and the yield, calculated on formaldehyde intake, amounted to 70%.

### Example 7

The experiment described in Example 4 was repeated using 8.2 g phenol as acid promoter. After 5 h the yield of glycol aldehyde amounted to 50%, calculated on formaldehyde intake, with a selectivity of 87%m.

### Example 8 (for comparison)

The experiment described in Example 5 was repeated but using 2.5 mmol triphenylphosphine instead of tri(p-chlorophenyl)phosphine. The selectivity amounted to only 54%m and the yield, calculated on formaldehyde intake, amounted to a mere 43%.

### Example 9 (for comparison)

The experiment described in Example 1 was repeated in the absence of acetic acid. The selectivity amounted to only 60%m and the yield, calculated on formaldehyde intake, did not exceed 5%.

### Claims

1. Process for the preparation of glycol aldehyde by reacting formaldehyde with hydrogen and carbon monoxide in the presence of a catalyst system derived from a rhodium-containing catalyst precursor and/or a cobalt-containing catalyst precursor, an acid and a promoter $XR_3$, X representing P, As or Sb and each R representing a similar or dissimilar substituted or unsubstituted hydrocarbyl group, characterized in that the reaction is carried out in the presence of an acid having a $pK_a \geqslant 3.5$ and that at least one R in the formula $XR_3$ represents an aryl group containing one or more electron-withdrawing groups.

2. Process according to claim 1, characterized in the use of aliphatic monocarboxylic acids, aliphatic dicarboxylic acids having at least two carbon atoms between the carboxylic moieties, aromatic carboxylic acids, partially esterified acids or solid organic acids or solid partially esterified polyacids.

3. Process according to claim 1 or 2, characterized in the use of formic acid, acetic acid, propionic acid, the butyric acids, or phenolic compounds.

4. Process according to any one of the preceding claims, characterized in the use of an acid having a $pK_a \geqslant 3.5$ in an amount of up to 50%v, preferably between 5%v and 30%v, in particular between 5% and 20%v.

5. Process according to any of the preceding claims, characterized in the use of rhodium and/or cobalt in an amount of between 0.001 to 10%, especially 0.01 to 5%, calculated as gram atoms of metal per mole of formaldehyde used as feedstock.

6. Process according to any one of the preceding claims, characterized in the use of promoters $PR_3$ containing halogen moieties in the meta or para position with respect to the phenyl group, trihalomethyl groups in the meta or para position, nitro groups in the meta or para position or alkoxy or aryloxy groups in the meta position.

7. Process according to claim 6, characterized in the use of promoters according to the general formula $XR_3$, wherein each group R is similar and contains at least one electron-withdrawing group.

8. Process according to claim 7, characterized in the use of tri(p-chlorophenyl)phosphine or tri(p-bromophenyl)phosphine.

9. Process according to any of claims 6—8, characterized in the use of a promoter to catalyst ratio in the range of from 1:1 to 100:1, especially 2:1 to 20:1, calculated as mol of promoter per gram atom of rhodium plus cobalt.

10. Process according to any one of the preceding claims, characterized in the use of carbon monoxide and hydrogen in a molar ratio in the range of from 30:70 to 80:20.

11. Process according to any one of the preceding claims, characterized in that the reaction is carried out at a temperature in the range of from 30 to 200°C.

12. Process according to any one of the preceding claims, characterized in the use of solvents having multiple bonds from carbon to other atoms, in particular N,N-disubstituted amides.

**Revendications**

1. Procédé de préparation d'un glycolaldéhyde par réaction du formaldéhyde avec l'hydrogène et l'oxyde de carbone en présence d'un système catalytique dérivé d'un précurseur de catalyseur contenant du rhodium et/ou un précurseur de catalyseur contenant du cobalt, un acide et un activant $XR_3$, X représentant P, As ou Sb et les R représentant chacun un groupe hydrocarbyle similaire ou différent, substitué ou non substitué, caractérisé en ce qu'on effectue la réaction en présence d'un acide ayant une valeur $pK_a \geqslant 3,5$ et en ce qu'au moins un R dans la formule $XR_3$ représente un radical aryle contenant un ou plusieurs groupes de soutirage d'électrons.

2. Procédé selon la revendication 1, caractérisé par l'utilisation d'acides aliphatiques monocarboxyliques, d'acides aliphatiques dicarboxyliques contenant au moins deux atomes de carbone entre les fragments carboxyliques, d'acides aromatiques carboxyliques, d'acides partiellement estérifiés ou d'acides organiques solides ou polyacides solides partiellement estérifiés.

3. Procédé selon la revendication 1 ou 2, caractérisé par l'utilisation d'acide formique, d'acide acétique, d'acide propionique, d'acides butyriques ou de composés phénoliques.

4. Procédé selon l'une quelconque des revendications précédentes, caractérisé par l'utilisation d'un acide ayant une valeur $pK_a \geqslant 3,5$ en une proportion jusqu'à 50% en volume, de préférence entre 5 et 30% vol. et, en particulier, entre 5 et 20% vol.

5. Procédé selon l'une quelconque des revendications précédentes, caractérisé par l'utilisation de rhodium et/ou de cobalt en une proportion de 0,001 à 10%, surtout 0,01 à 5%, calculée en atomes-g de métal par mole de formaldéhyde servant de charge.

6. Procédé selon l'une quelconque des revendications précédentes, caractérisé par l'utilisation d'activants $PR_3$ contenant des fragments d'halogène en position méta ou para par rapport au groupe phényle, des radicaux trihalométhyle en position méta ou para, des radicaux nitro en position méta ou para ou des radicaux alcoxy ou aryloxy en position méta.

7. Procédé selon la revendication 6, caractérisé par l'utilisation d'activants de formule générale $XR_3$ dans laquelle les radicaux R sont similaires et chacun contient au moins un groupe de soutirage d'électrons.

8. Procédé selon la revendication 7, caractérisé par l'utilisation de tri(p-chlorophényl)phosphine ou de tri(p-bromophényl)phosphine.

9. Procédé selon l'une quelconque des revendications 6 à 8, caractérisé par l'utilisation d'un rapport d'activant au catalyseur compris entre 1:1 et 100:1, surtout entre 2:1 et 20:1, calculé en moles d'activant par atomes-g de rhodium- + cobalt.

10. Procédé selon l'une quelconque des revendications précédentes, caractérisé par l'utilisation d'oxyde de carbone et d'hydrogène dans un rapport molaire de 30:70 à 80:20.

11. Procédé selon l'une quelconque des revendications précédentes, caractérisé en ce qu'on effectue la réaction à une température de 30 à 200°C.

12. Procédé selon l'une quelconque des revendications précédentes, caractérisé par l'utilisation de solvants ayant des liaisons multiples du carbone à d'autres atomes, en particulier d'amides N,N-disubstitués.

**Patentansprüche**

1. Verfahren zur Herstellung von Glykolaldehyd durch Umsetzung von Formaldehyd mit Wasserstoff und Kohlenmonoxid in Gegenwart eines Katalysatorsystems, abgeleitet von einem Rhodium-haltigen Katalysatorvorläufer und/oder einem Cobalt-haltigen Katalysatorvorläufer, einer Säure und einem Promotor $XR_3$, wobei X Phosphor, Arsen oder Antimon und R gleiche oder unterschiedliche substituierte oder unsubstituierte Kohlenwasserstoffgruppen bedeuten, dadurch gekennzeichnet, daß die Reaktion in Gegenwart einer Säure mit $pK_a \geqq 3,5$ durchgeführt wird und daß zumindest ein Substituent R des Promotors $XR_3$ eine Arylgruppe mit einer oder mehreren Elektronenabziehenden Gruppen ist.

2. Verfahren nach Anspruch 1, dadurch gekennzeichnet, daß man aliphatische Mono- oder Dicarbonsäure mitzumindest 2 Kohlenstoffatomen zwischen den Säuregruppen, aromatische Carbonsäure, teilweise veresterte Säuren oder feste organische Säuren oder feste teilveresterte Polysäuren verwendet.

3. Verfahren nach Anspruch 1 oder 2, dadurch gekennzeichnet, daß man Ameisen-, Essig-, Propionsäure, Buttersäuren oder phenolische Verbindungen verwendet.

4. Verfahren nach einem der vorhergehenden Ansprüche, dadurch gekennzeichnet, daß man eine Säure mit $pK_a \geqq 3,5$ in einer Menge bis zu 50 Vol.-%, vor zugsweise 5 bis 30 Vol.-%, insbesondere 5 bis 20 Vol.-%, verwendet.

5. Verfahren nach einem der vorhergehenden Ansprüche, dadurch gekennzeichnet, daß man Rhodium und/oder Cobalt in einer Menge von 0,001 bis 10%, insbesondere 0,01 bis 5% — berechnet als g-Atom — Metall je Mol Formaledhyd des Ausgangsmaterials verwendet.

6. Verfahren nach einem der vorhergehenden Ansprüche, dadurch gekennzeichnet, daß man als Promotor $PR_3$ einen solchen verwendet, der in Meta- oder Parastellung zur Phenylgruppe, Halogen-haltige Gruppen, Trihalogenmethylgruppen oder Nitrogruppen oder in Metastellung Alkoxy-oder Aryloxygruppen aufweist.

7. Verfahren nach Anspruch 6, dadurch gekennzeichnet, daß man einen Promotor $XR_3$ verwendet, dessen Substituenten R gleich sind und zumindest eine Elektronen abziehende Gruppe enthalten.

8. Verfahren nach Anspruch 7, dadurch gekennzeichnet, daß man Tri(p-chlorphenyl)phosphin oder Tri(p-bromphenyl)phosphin verwendet.

9. Verfahren nach einem der Ansprüche 6 bis 8, dadurch gekennzeichnet, daß man ein Verhältnis Promotor zu Katalysator 1:1 bis 100:1, vorzugsweise 2:1 bis 20:1, einhält, berechnet als Mol Promotor je g-Atom Rhodium + Cobalt.

10. Verfahren nach einem der vorhergehenden Ansprüche, dadurch gekennzeichnet, daß man Kohlenmonoxid und Wasserstoff in einem Molverhältnis 30:70 bis 80:20 verwendet.

11. Verfahren nach einem der vorhergehenden Ansprüche, dadurch gekennzeichnet, daß man bei einer Temperatur von 30 bis 200°C arbeitet.

12. Verfahren nach einem der vorhergehenden Ansprüche, dadurch gekennzeichnet, daß man ein Lösungsmittel mit Doppelbindungen zwischen Kohlenstoff und anderen Atomen verwendet, insbesondere N,N-disubstituierte Amide.